(19) Europäisches Patentamt / European Patent Office / Office européen des brevets

(11) **EP 2 241 344 B1**

(12) **EUROPEAN PATENT SPECIFICATION**

(45) Date of publication and mention
of the grant of the patent:
**11.12.2013 Bulletin 2013/50**

(51) Int Cl.:
**A61M 5/168** (2006.01)

(21) Application number: **09005407.3**

(22) Date of filing: **16.04.2009**

(54) **Ambulatory infusion device with sensor testing unit**

Tragbare Infusionsvorrichtung mit Sensortesteinheit

Dispositif de perfusion ambulatoire avec unité de test de capteur

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(43) Date of publication of application:
**20.10.2010 Bulletin 2010/42**

(73) Proprietors:
• **F. Hoffmann-La Roche AG
4070 Basel (CH)**
Designated Contracting States:
**AT BE BG CH CY CZ DK EE ES FI FR GB GR HR
HU IE IS IT LI LT LU LV MC MK MT NL NO PL PT
RO SE SI SK TR**
• **Roche Diagnostics GmbH
68305 Mannheim (DE)**
Designated Contracting States:
**DE**

(72) Inventors:
• **Lindegger, Stefan
4950 Huttwil (CH)**
• **Schrotberger, Reto
3012 Bern (CH)**
• **Müri, Alex
3097 Liebefeld (CH)**

(74) Representative: **Rentsch Partner AG
Rechtsanwälte und Patentanwälte
Fraumünsterstrasse 9
Postfach 2441
8022 Zürich (CH)**

(56) References cited:
**EP-A- 1 649 884      EP-B1- 0 416 911
EP-B1- 1 652 542**

**Description**

The present invention is directed towards an ambulatory infusion device for the infusion of a liquid drug into a patient's body over an extended time period. The infusion device comprises a sensor testing unit for verifying correct operation of a sensor assembly of the infusion device. The invention is further directed towards a corresponding method for verifying correct operation of a sensor assembly.

Ambulatory infusion devices for the infusion of a liquid drug over an extended time period are known in the art for a number of therapies. In particular, such devices form the basis for a state-of-the-art therapy of Diabetes Mellitus by CSII (Continuous Subcutaneous Insulin Infusion). Such an ambulatory infusion device is disclosed, for example, in the WO2003053498A2 to which reference is made for the general design and features of such devices according to the state of the art. In the following, an ambulatory infusion device according to the technical field as stated above and in particular an ambulatory infusion device in accordance with the present invention is referred to as "device".

Besides diabetes therapy, those devices may be used for a number of further therapies, such as cancer treatment or pain therapy, without requiring substantial modification. The following description of the present invention mainly refers to CSII therapy without limiting the invention to this specific application.

For generally supervising the device operation and for detecting the occurrence of hazardous situations, such as fluidic leakages and occlusions, typical state-of-the-art devices comprise one or multiple sensors. Devices of the syringe-driver type which are the currently most common design typically comprise a force sensor as part of the drive system which measures the reaction force that is excreted by the drive system onto the plug of a drug cartridge. Alternatively or additionally, they may comprise a fluidic pressure sensor, for example at the drug cartridge outlet or directly at the infusion site, or a drug flow sensor. Since the major purpose of those sensors is the early detection of occlusions, i.e., blockages of the infusion line, they are also referred to as 'occlusion sensors'.

Correct operation of such sensors is important for the overall system and patient safety. Typically the sensor is located at a position remote from the other electronics components and therefore requires cabling, for example via a flexible printed circuit board. In some embodiments, the sensor may be a disposable sensor which is connected via releasable electrical connectors. Since wiring and connectors are known to be especially critical it is particularly desirable to assure their integrity and to safely detect interruptions, loose contacts, short circuits, and the like.

The patent application US 2007/0149926 A1 discloses fault detection in a syringe-driver device by correlating independent force measures. In particular it is considered if a variation in the motor current which is indicative for a variation in the reaction force is also reflected in a force signal which is measured by a force sensor. This approach is linked to specific hardware architectures and is dependent on a considerable number of influence parameters.

Document EP-A-1 649 884 describes an infusion device comprising a sensor according to the preamble of claim 1.

It is the overall objective of the present invention to improve the safety of ambulatory infusion devices and to ensure reliable detection of device defectives for a large number of device architectures and at low costs.

This overall goal is achieved based on the on the insight that some sensors which are typically used in ambulatory infusion devices, in particular occlusion sensors and the subsequent measurement circuitry they are connected to, provide output variables in dependence of a supply variable which is required for operating the sensor. In accordance with the invention, correct operation of the sensor assembly is verified by varying the supply variable and detecting if this variation is correctly reflected by the output variable.

According to one aspect of the invention, the goal of improving the safety of ambulatory infusion devices is achieved by providing an ambulatory infusion device as defined in the preamble of Claim 1. The device comprises:

a) a sensor assembly with a sensor, the sensor assembly being configured to provide an output variable in dependence of an infusion variable and in dependence of a supply variable,
b) a supply unit, the supply unit being coupled to the sensor and being configured to provide the supply variable,
c) a sensor testing unit for verifying correct operation of the sensor assembly, the sensor testing unit being coupled to the sensor assembly and the supply unit, the sensor testing unit being configured to carry out a sensor testing sequence, the sensor testing sequence comprising the steps of controlling the supply unit to vary the supply variable and to determine whether the variation of the supply variable is reflected by an expected variation of the output variable.

In the framework of this invention, the term 'variable' refers to a physical variable, such as pressure, force, current, ohmic resistance, and the like. It further refers to the numeric representation of such a physical variable, as obtained, for example, by analogue-to-digital conversion of such a variable. The value of a variable may be constant or varying over time. For simplification purposes, a specific value of a variable may also be referred to as 'variable' if the meaning is obvious within the context.

The term 'infusion variable' refers to a physical variable which is measured by the sensor of the sensor assembly and which is associated with the drug administration. The infusion variable may especially be a drug pressure or a variable which is correlated with the drug pressure, such as the reaction force exerted by a drive system of the device.

The term 'sensor assembly' refers to an electrical sensor for a physical variable, in particular an infusion variable, and the measurement circuitry which is typically provided for filtering, amplification, analogue-to-digital conversion, and the like. The sensor may especially be an occlusion sensor as described above.

The term 'output variable' refers to the variable which is generated as the overall output of the sensor assembly, such as a number representing a digitalized voltage output. The term 'sensor output variable' refers to an electrical variable which is generated or varied by the sensor as such in dependence of the infusion variable. Exemplary sensor output variables are the current through a force-sensitive resistor (FSR) at constant voltage or the differential voltage of a resistive strain-gauge bridge under mechanical load.

In case the sensor testing unit detects that a variation of the supply variable is not reflected by a corresponding and expected variation of the output variable, the presence of a fault in the sensor assembly can be assumed. The device is advantageously configured to carry out fault handling steps such as stopping the drug administration and alerting the user, for example by an optical, acoustical, and/or tactile alert.

The device may be configured to initiate fault handling steps if the presence of a fault is indicated in a single sensor testing sequence. However, since temporary distortions may occur due to mechanical effects such as shocks or vibrations and/or due to electrical effects such as electromagnetic interferences, the device may be configured to assume the presence of a fault only if it is indicated in a number of testing sequences. The sensor testing unit may especially be configured to repeat the sensor testing sequence once or repeatedly in case a first sensor testing sequence indicates the presence of a fault.

The expected variation of the output variable upon a variation of the supply variable is determined by the design of the sensor and its physical operation principle as well as the subsequent measuring circuitry as will be understood in more detail as the description proceeds.

In some embodiments, the sensor assembly comprises a sensor with at least one force-sensitive and/or pressure-sensitive resistor and/or at least one strain gauge. A force-sensitive resistor (FSR) may for example be coupled to the drive system of the infusion device, for example according to the disclosure of US6485465B2. Alternatively, a bending beam and strain gauge arrangement may be used for force measurement. In some embodiments, the sensor assembly comprises a strain-gauge measurement bridge for measuring the reaction force. A corresponding design is disclosed in WO2001/24854A1. The sensor is advantageously mechanically and/or electrically pre-loaded to always ensure a non-vanishing sensor output signal.

In some embodiments, the supply unit comprises a variable voltage supply or a variable current supply, in dependence of the sensor. Accordingly, the supply variable may especially be a supply current if the sensor is designed for controlled current supply or a supply voltage if the sensor is configured for controlled voltage supply. Both kinds of sensors are known in the art and are commercially available in a large variety.

The supply unit may be configured to provide a set of at least two discrete levels of the supply variable, such as different supply voltage levels or supply current levels. This may be achieved by various means such as at least two supplies. It may alternatively be achieved by a combination of one supply and additional resistors, such as dropping resistors or shunts, and a supply selector which is controlled by the sensor testing unit for switching between the levels in order to vary the supply variable. The supply selector is advantageously realized as solid state circuit.

In some embodiments, the supply unit is configured to provide two discrete levels of the supply variable, namely a testing supply level and an operating supply level. The operating supply level is the standard supply level for which the sensor is designed and which is normally used for measuring the infusion variable. For this type of embodiment, the sensor testing sequence comprises switching the supply variable between the two discrete supply levels once or repeatedly. The corresponding course of the supply variable is a step or a square wave.

Alternatively to two supply levels, the supply unit may be configured to provide a set of more than two discrete supply levels and the sensor testing sequence comprises switching between the single supply levels for varying the supply variable. In a further variant, the supply unit may be configured to provide a continuously varying supply variable, for example of the sinusoidal wave type or the triangle wave type. In the following, two discrete supply levels are generally assumed. The modifications required for a set of more than two discrete supply levels or for a continuously varying supply variable are straight forward.

In some embodiments, the expected variation of the output variable upon a variation of the supply variable is proportional to the variation of the supply variable. Several types of sensors provide a sensor output variable which is directly or inverse proportional the level of the supply variable. This is the case, for example for the current through an FSR as sensor output variable with the FSR being supplied by a given voltage, or for the voltage drop over an FSR as sensor output signal with the FSR being supplied by a given current. It further holds true for the differential voltage of a sensor bridge as sensor output variable with the sensor bridge being supplied by a given voltage or current. The same relation further holds true for the rectified voltages and currents of capacitive or inductive sensors or sensor bridges if supplied by a sinusoidal AC supply.

If the sensor output variable is proportional to a supply variable, this also holds true for the output variable, assuming the measurement circuitry is linear. In case of an offset, the absolute value of the output variable may not be proportional to the absolute value of the supply variable. The expected variation of the output variable, however, is proportional to the variation of the supply signal as long as the  characteristic is linear. A substantially non-linear characteristic of the sensor may be compensated in the measurement circuitry by applying linearization techniques known in the art.

As will become more readily apparent below in the framework of exemplary embodiments, an expected proportional relation allows the sensor testing unit to determine if the variation of the supply variable is reflected by the expected variation of the output variable by simple division operations.

In some embodiments, the sensor testing unit is configured to measure the value of the supply variable. Measuring the actual value of the supply variable is advantageous for determining the relation between a variation of the supply variable and the output variable if the supply variable is subject of uncertainties and variations, such as the voltage supplied by a battery and/or if the supply variable is influenced by the sensor. This is the case, for example, for a supply voltage which is supplied to an FSR by a non-ideal voltage supply of non-negligible impedance.

Further aspects and favorable embodiments of a device according to the present invention will become more apparent from the exemplary embodiments as discussed below in more detail.

According to another aspect of the invention, the goal of improving the safety of ambulatory infusion devices is achieved by providing a method for verifying correct operation of a sensor assembly of the ambulatory infusion device by carrying out a sensor testing sequence. The sensor assembly is configured to provide an output variable in dependence of an infusion variable and in dependence of a supply variable provided to a sensor of the sensor assembly. The sensor testing sequence comprises the steps of:

> a) varying the supply variable and
> b) determining if the variation of supply variable is reflected by an expected variation of the output variable.

Advantageously, the method further comprises the step of sampling the output variable at different levels of the supply variable.

In some embodiments, varying the supply variable is carried out by switching the value of the supply variable between at least two discrete levels. The supply variable may especially be a supply voltage or a supply current. The switching may be performed by selecting either of at least two independent supplies or by varying a  single supply as described above in the framework of a device according to the invention. Alternatively, the sensor testing sequence may comprise varying the supply variable substantially continuously by providing, for example a sinusoidal wave or triangle wave voltage or current supply.

In some embodiments, the expected variation of the output variable upon a variation of the supply variable is proportional to the variation of the supply variable. For those embodiments, the sensor testing sequence comprises determining if the variation of the output variable is proportional to the variation of the supply variable.

In favourable embodiments, the sensor testing sequence comprises varying the supply variable such that the expected variation of the output variable is large as compared to a variation of the output variable resulting from a variation of the infusion variable while carrying out the sensor testing sequence. This implies that the variation of the supply variable should be large and that the steps of varying the supply variable and sampling the output variable should be carried out in a time interval which is short as compared to the expected variation of the measurement signal.

For noise reducing purposes, the sensor testing may further comprise repeatedly varying the supply variable and sampling the output variable as will be described below in more detail.

For improving the robustness, it may further or alternatively be advantageous to expect the variation of the output signal to be in an expected range rather than having a precisely defined value. The width of such an expected range depends on the expected level of noise and/or distortions. For such an embodiment, the variation of the output signal is an expected variation if it is within the expected range.

In some embodiments, sensor testing is carried out in defined time intervals and/or prior to and/or during and/or after drug administration by the infusion device. The defined time intervals are advantageously short enough to detect a fault of the sensor assembly without significant delay, for example every second, every minute, or every three minutes. For devices which do not administer the drug continuously but are configured to administer drug boli on demand and/or to administer drug substantially continuously in pulsed way with a drug pulse being administered with time intervals of several minutes, sensor testing is advantageously carried out prior to and/or after the drug administration.

If larger drug amounts, such as drug boli, are administered by administering separated drug pulses, a sensor testing sequence may additionally or alternatively be carried out between the administrations of the single drug pulses.

Further aspects and embodiments of the method may be derived in a straight-forward way from the embodiments of a device according to the invention as described above and from exemplary embodiments as described below.

In the following, exemplary embodiments of the invention are described in more detail with reference to the figures.

Figure 1 shows the structural view of an exemplary device according to the invention.

Figure 2 exemplary shows a course of the supply variable and of the output variable as functions of time for a device in accordance with Figure 1.

Figure 3 exemplary shows a further course of the supply variable and of the output variable as well as a value computed from these variables as functions of time for another device in accordance with Figure 1.

Figure 4 shows a more detailed structural view of an exemplary device in accordance with Figure 1.

Figure 5 shows a schematic view of a further exemplary device in accordance with Figure 1.

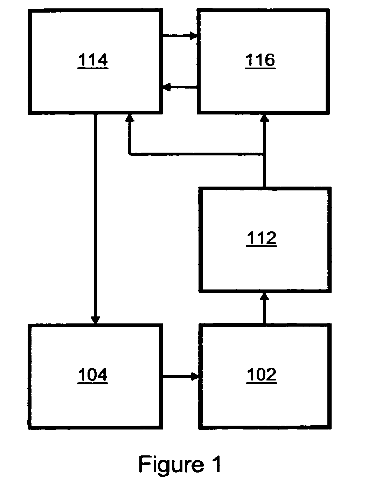

Figure 1 schematically shows an exemplary embodiment of an infusion device according to the present invention with a sensor 102, a supply unit 104, measuring circuitry 112, a sensor testing unit 114 and a controller unit 116 of the infusion device. The sensor 102 and the measuring circuitry 112, in combination, form the sensor assembly. The controller unit 116 controls and monitors the operation of the device. It is an electronic controller and typically includes state-of-the-art components such as one or multiple micro controllers, memory, clock circuitry, interface circuitry, and the like. Further components of the infusion device such as a drive unit, a drug cartridge, a power supply, a user interface, and the like, are not shown in Figure 1 but are obvious for a person skilled in the art. The overall design of the infusion device may, for example, be in accordance with the device as disclosed in WO2003053498A2 or any commercially insulin infusion pump.

The characteristics of the sensor 102 are in the following assumed to be such that the sensor output variable is proportional to a supply variable. It is further assumed that the transfer characteristics of the measurement circuitry 112 are linear, such that the output variable of the sensor assembly is expected to be proportional to the supply variable. None of these assumptions, however, is required as long as the corresponding relation is unique.

The supply unit 104 is configured to supply the sensor 102 with a varying supply variable, such as different supply voltage levels or supply current levels. The output variable is sampled and pre-processed by the measuring circuitry 112. The measuring circuitry 112 typically comprises pre-processing circuitry such as amplifiers and/or signal conditioning circuitry, a sampling-and-hold circuit and an analogue-to-digital converter as known in the art.

The measuring circuitry 112 feeds the output variable into the sensor testing unit 114 as well as the controller unit 116. The general evaluation of the output variable for the administration supervision and the detection of administration hazards is performed by the controller unit 116.

For testing the sensor assembly, the sensor testing unit 114 controls the supply unit 104 to vary the supply variable and detects whether or not the variation of the output variable reflects the variation of the supply variable. If this is not the case, the sensor testing unit 114 sends a corresponding fault signal to the controller unit 116 which initiates further fault handling steps such as terminating the drug administration and alarming the user via a user interface, in particular by at least one of an optical, acoustical, and tactile signal.

Testing of the sensor assembly is initiated by the controller unit 116 and is carried out in fixed time intervals, for example every 1 min. or every 3min. In addition or alternatively, it may be carried out before, during and/or or after a drug administration.

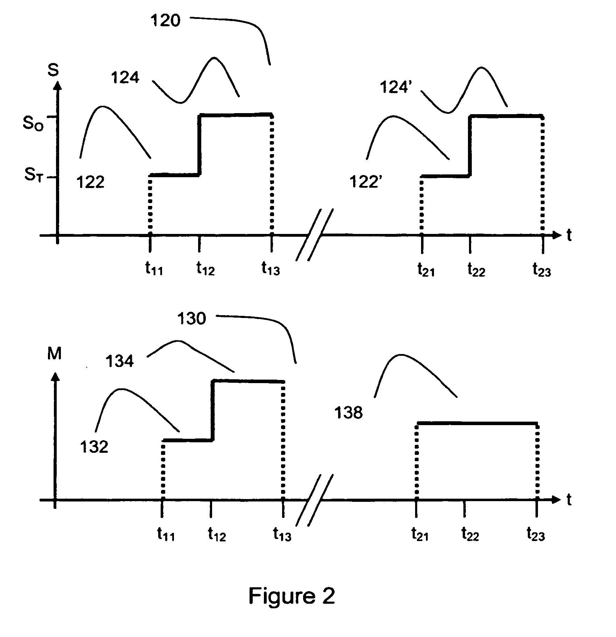

In the following, a sensor testing sequence is described in more detail with additional reference to Figure 2. Figure 2 exemplarily shows the course 120 of the supply variable S and the course 130 of the output variable M as functions of time t. It is assumed that the supply unit 104 may provide two discrete levels of the supply variable S, namely a testing supply level $S_T$ and an operating supply level So, with the operating supply level So being the nominal supply level for which the sensor 102 is designed. In addition, the supply unit 104 may not provide a supply variable, corresponding to the sensor 102 being inactive. Without loss of generality it is further assumed that the testing supply level $S_T$ is half of the operating supply level So.

In an initial state, the supply unit 104 does not provide a supply variable, resulting in the sensor 102 being inactive. At a first point in time, $t_{11}$, the supply unit 104 is controlled to provide the supply variable S with the testing supply level $S_T$, as indicated by the curve section 122. The resulting testing output value $M_T$ is indicated by the curve section 132 and is sampled by the measuring circuitry 112. At the second point in time, $t_{12}$, the supply unit 104 is controlled to provide the supply variable S with the operating supply level So as indicated by the curve section 124 and the corresponding operating output value $M_o$ as given by curve section 134 is sampled by the measuring circuitry 112.

The two sampled values $M_T$ and $M_o$ of the output variable are evaluated by the sensor testing unit 114. Therefore, the sensor testing unit 114 checks if the relation

$$\frac{M_T}{S_T} = \frac{M_O}{S_O}$$

$$\frac{M_T}{M_O} = \frac{S_T}{S_O} \qquad (1)$$

is approximately met, with (1) reflecting the expected proportional relation between the supply variable and the output variable.

As can be seen from the curve 130, the change of the level of the supply variable S at time $t_{12}$ is reflected by a proportional change of the level of the output variable M, thus indicating correct operation of the sensor 102 and the measuring circuitry 112. The sensor 102 is deactivated at time $t_{13}$.

After switching the supply from the testing supply level $S_T$ to the operating supply level So at time $t_{12}$, a drug administration may be performed in the time interval from $t_{12}$ to $t_{13}$, that is, with the sensor 102 being operated at its nominal supply level. Depending on the specific administration regime of the infusion device, the sensor 102 may be operated continuously during the administration or may be operated in a non-continuous sequence. Additionally or alternatively, the sensor testing sequence may be carried out repeatedly during drug administration.

The sensor testing sequence starting at time $t_{21}$ and ending at the point tin time $t_{23}$ shows a situation where sensor 102 and/or the measuring circuitry 112 are defective. As indicated by the curve sections 122', 124', the sensor 102 is consecutively supplied with the testing supply level $S_T$ at time $t_{21}$ and the operating supply level So at time $t_{22}$ as described above. However, the output variable M does not reflect this variation of the supply voltage, but stays constant, as indicated by the curve section 138. For other types of faults of the sensor assembly, the output variable M shows a variation, in particular noise, which does not reflect the variation of the supply variable.

The proportional relation between the supply variable S and the output variable M according to (1) is based on a number of assumptions and idealizations. In particular, it is assumed that the infusion variable such as a mechanical load which is acting on the sensor 102 stays constant during the sensor testing sequence. Furthermore, it is assumed that neither the sensor output variable nor the output variable M has an offset component, no noise or drift are present, and the like.

For taking distorting effects into account, the sensor testing unit 114 may test for the ratio of the output variable at the different levels of supply variables to be in a given expected range, the width of the range reflecting the admissible amount of distortion. This width of the expected range may be selected quite liberal in order to avoid false alarms as long as it is small as compared to the variation that can be expected based on the variation of the supply variable S. Further more advanced methods for considering noise, such as a statistical hypothesis testing for a given ratio or difference of the output variables may be employed as well if required.

The time delay between taking the samples of the output variable should be short in order to minimize the superimposed effect of a variation of the infusion variable. For the example shown in Figure 2, the sampling of the output variable M may be performed shortly before $t_{12}$ and $t_{22}$ for the testing supply level $S_T$ and shortly after $t_{12}$ and $t_{22}$ for the operating supply level So.

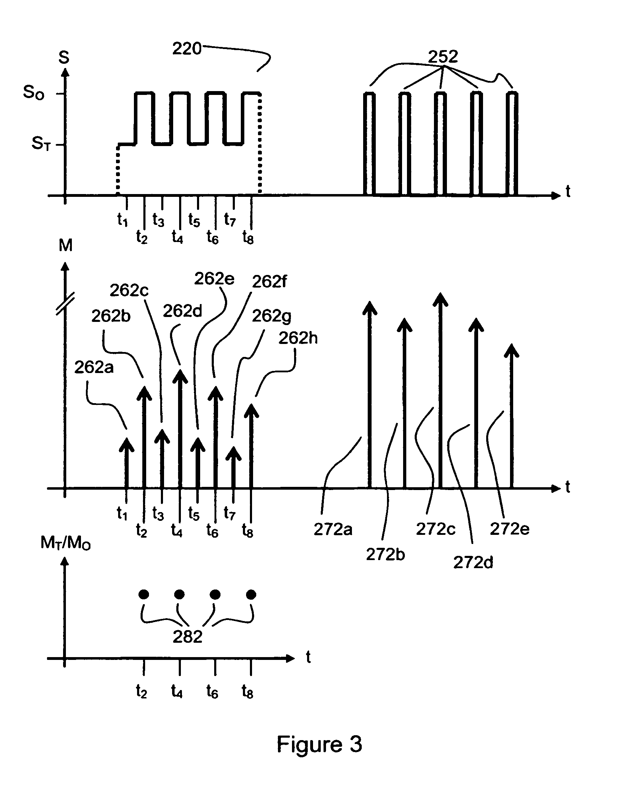

The curves shown in Figure 3 and the hardware structure shown in Figure 1, in combination, illustrate a further exemplary embodiment of the invention. In this embodiment, the robustness is increased by averaging over multiple samples.

The curve 220 in Figure 3 illustrates the supply variable S as a function of time t. The supply unit 104 is controlled to repeatedly supply the sensor 102 with the testing supply level $S_T$ followed by supplying the sensor 102 with the operating supply level So. This results in an overall square wave signal with the times $t_1, t_2, ..., t_8$ indicating the middle of each segment. The output variable M is sampled at the times $t_1, t_2, ..., t_8$, as shown by sampling arrows 262a, 262b, ... 262h in the middle diagram of Figure 3. The values sampled at the times $t_1, t_3, t_5, t_7$ are testing output values $M_T$ while the values sampled at the times $t_2, t_4, t_6, t_8$ are operating output values $M_O$. Here and in the following, arrows in a time diagram indicate a sampling with the length of the arrow corresponding to the sampled value.

It can be seen that the sampled values of the output variable M generally reflect the variation of the supply variable S. However, the value of the output variable M is not constant at either of the supply variable levels, but shows some variation due to noise and distortions.

The values of the output variable M sampled in neighboring segments of the supply variable S are evaluated pair wise. Therefore, the sensor testing unit 114 may compute an average of the $M_T/M_O$ quotients computed according to

| $t$ | $t_2$ | $t_4$ | $t_6$ | $t_8$ |
|---|---|---|---|---|
| $M_T/m_0$ | $M(t_1)/M(t_2)$ | $M(t_3)/M(t_4)$ | $M(t_5)/M(t_6)$ | $M(t_7)/M(t_8)$ |

and use the average $M_T/M_O$ quotient in *(1)*.

Because many distorting effects and in particular variations of the infusion variable occur slow in comparison with switching the level of the supply variable S and because taking neighboring samples of the output variable M, the ratio $M_T/M_O$ is largely independent of those effects as shown by the dots 282 in the lowest diagram of Figure 3. The dots indicate the computed quotients $M_T/M_O$ according to the table given above.

In a further improved embodiment, the ratios $M(t_1)/M(t_2)$, $M(t_3)/M(t_2)$, $M(t_3)/M(t_4)$, ... are computed. That is, except the outermost samples, each sampled value of the output variable M is used for the computation of two $M_T/M_O$ ratios.

Further more advanced filtering and distortion reduction techniques may be used as well, but are typically not required.

As further illustrated in Figure 3, a series of five consecutive drug pulses is administered by the infusion device after completing the sensor testing sequence. For the administration of each of the drug pulses, the sensor 102 is temporarily supplied with the operating supply level So as indicated by the pulses 252. During or after completing the administration of each drug pulse, the infusion variable is determined by sampling the output variable M, as indicated by the sampling arrows 272a, 272b ... 272d, followed by an evaluation in the controller unit 116. The shown variation of the sampled measurement currents is indicative for a corresponding typical variation of the infusion variable, particular of the force or pressure which is acting on the sensor 102 during administration.

In the exemplary diagrams of Figure 2 and Figure 3, the testing supply level $S_T$ is half of the operating supply level So. Accordingly, the expected testing output value $M_T$ is half of the operating output value $M_o$. It will the appreciated by a person skilled in the art that other ratios of the testing supply level $S_T$ and the operating supply level So may be used as well and that the testing supply level $S_T$ may also be higher than the operating supply level So. It will further be appreciated that the device and the testing method may be modified in a straight-forward way for more than two levels of the supply signal or for a continuous variation of the supply signal.

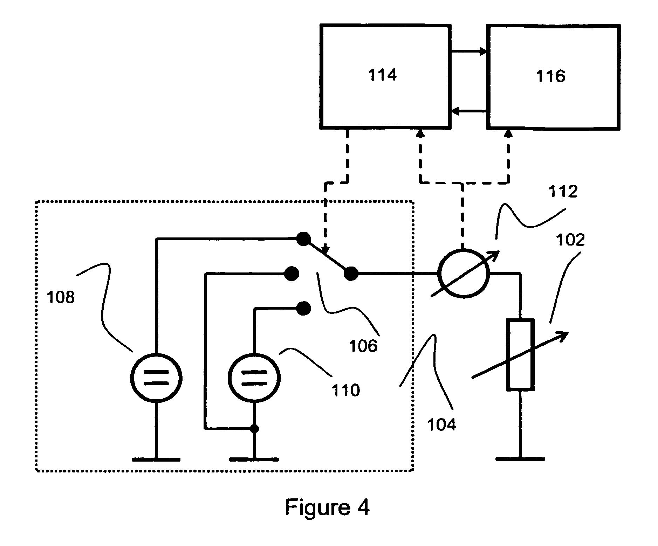

In Figure 4, a more detailed view of an exemplary hardware structure for an embodiment according to Figure 1 is shown. The sensor 102 is an FSR which measures the reaction force exerted onto the drug in the drug container as disclosed, for example, in US6485465B2 Alternatively, it may be a pressure sensitive resistor which measures the fluidic pressure of the drug at the outlet of the drug cartridge or may be a strain gauge which is mounted onto a bending beam. In this embodiment, the supply voltage U which is supplied to the sensor 102 is the supply variable S and the resulting current I through the sensor 102 is the sensor output variable. The measuring circuitry 112 may especially comprise a current-to-voltage converter followed by an Analogue-to-Digital Converter (ADC), such that the output signal M is a number which is proportional the current through the sensor 102 if the sensor assembly operates correctly. For a given value of the infusion variable, that is, the mechanical load which is acting on the sensor 102, and thus a given ohmic resistance of the sensor 102, the current is proportional to the supply voltage.

The resistance of the sensor 102 may be directly or reciprocal proportional to the acting force or pressure or have any defined non-linear characteristics.

The supply unit 104 comprises a testing voltage supply 108 for providing a testing supply voltage $U_T$ and a operating voltage supply 110 for providing a operating supply voltage $U_O$. These voltages correspond to the testing supply level $S_T$ and the operating supply level So as described above. The supply unit 104 further comprises a supply selector 106 for alternatively connecting the sensor 102 to either of the testing voltage supply 108, the operating voltage supply 110 or ground.

The voltage supplies 108, 110 may be two different voltage supplies as shown in Figure 4 or may alternatively be implemented as one variable voltage supply. In the latter case, the sensor testing unit 114 controls, via the supply selector 106, the voltage supply to be either of the testing supply voltage $U_T$ or the operating supply voltage, $U_o$, respectively.

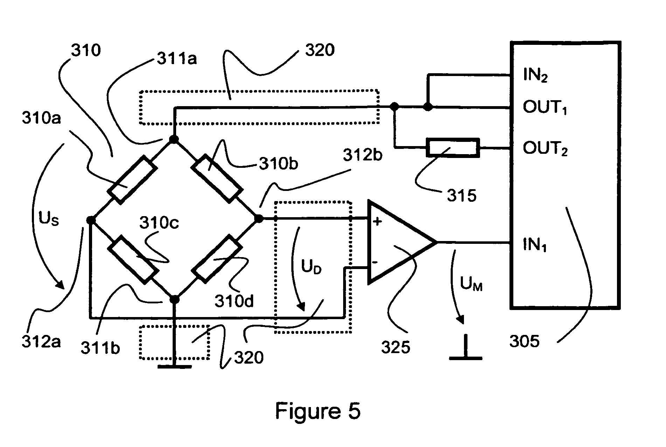

The testing supply voltage $U_T$ and the operating supply voltage $U_o$ are selected in accordance with the sensor specification such that the sensor output is always a valid input for the measurement circuit 112. In a typical embodiment, the measurement circuit 112 comprises an operational amplifier and an ADC with an unsymmetrical supply, i.e., a generally positive voltage supply with respect to ground, as described below in more detail with reference to Figure 5. In this case, the amplifier and the ADC can only process positive voltage inputs within a certain range which should in particular be neither zero nor negative. For this type of embodiment, a testing supply voltage $U_T$ which is in a range of 1/3 to 2/3 of the operating supply voltage $U_o$ is favored. If the measurement circuitry in contrast is supplied with a symmetric supply voltage with respect to ground and may process zero or negative input signals, the testing supply voltage $U_T$ may also be zero or negative.

Instead of the constant voltage supplies 108, 110, current supplies may be used to alternatively supply the sensor 102 with a testing supply current and an operating supply current. In this case, the voltage drop over the sensor 102 is used as sensor output variable and the measuring circuitry 112 is configured to sample and measure this voltage drop. For selecting the operating supply level and the testing supply level, the same considerations as given for a voltage supply hold true.

Figure 5 schematically shows the hardware structure of a further exemplary device in accordance with the invention. With respect to the operation, reference is additionally made to Figure 2 and Figure 3 as well as the corresponding description as given above.

The senor of this embodiment is realized as resistive strain-gauge bridge 310 which comprises the single resistors 310a, 310b, 310c, 310d of resistances $R_a$, $R_b$, $R_c$, $R_d$. The resistors are assembled on a bending beam carrier by adhesive bonding or thick-film technologies The sensor bridge 310 is supplied via a pair of opposed connectors 311a, 311b with a supply voltage $U_S$. The differential voltage $U_D$ which can be measured between the other pair of opposed connectors 312a, 312b is the sensor output variable. The overall bridge resistance $R_B$ between the supply connectors 311a, 311b is typically in a range of 5k$\Omega$ to 100k$\Omega$.

The ambulatory infusion device is of the syringe-driver type and the resistors 310a, 310b, 310d, 310d are part of a strain-gauge beam arrangement which measures the reaction force which is excreted by a drive system onto the plug of a drug cartridge in the infusion device. A corresponding mechanical structure is exemplarily disclosed in WO2001/24854A1. Alternatively, the sensor bridge 310 is part of a fluidic drug pressure sensor. Advantageously, the sensor bridge 310 is mechanically pre-loaded.

The device further comprises a micro controller 305 which integrates major components of the controller unit of the infusion device, the sensor testing unit as well as part of the supply unit and the measurement circuitry as will become more readily visible in the following.

The single resistors 310a, 310b, 310d, 310d are arranged such that the bridge resistance $R_B$ which can be measured between the supply connectors 311a, 311b stays substantially constant while the single resistances $R_a$, $R_b$, $R_c$, $R_d$ depend on the mechanical load which is acting on the sensor. For this arrangement, the ratio $U_D/U_S$ of the differential voltage $U_D$ as sensor output variable and the supply voltage $U_S$ as the supply variable is proportional to mechanical load which is acting on the bridge, independent of the absolute value of the supply voltage $U_S$.

The differential voltage $U_D$ is amplified by an amplifier circuit 325 which is advantageously designed on the basis of an integrated instrumentation amplifier or a set of single operational amplifiers. The output of the amplifier 325 forms an output voltage $U_M$.

The output voltage $U_M$ is fed into an analogue input $IN_1$ of the micro controller 305 and is sampled and digitalized by an ADC, for example a 12Bit ADC which is integral with the micro controller 305. The output of the ADC forms the output variable M.

The micro controller 305 comprises two voltage output ports $OUT_1$, $OUT_2$, via which the sensor bridge 310 may alternatively supplied. If either of the outputs $OUT_1$, $OUT_2$ is active and provides an output voltage, the other of the two outputs is reconfigured to be in an inactive high-impedance state. Alternatively, it may be reconfigured as input port.

The two output ports $OUT_1$, $OUT_2$ provide the same standard supply voltage $U_0$ which is given by or derived from the power supply voltage of the infusion device and may, for example, be about 3 V for typical state-of-the-art technology. If the output port $OUT_1$ is active, the standard supply voltage $U_0$ is directly connected to the sensor bridge 310 as operating supply voltage $U_O$. If the output port $OUT_2$ is active, the standard supply voltage $U_0$ is connected via an additional dropping resistor 315 of constant resistance $R_D$ as testing supply voltage $U_T$. The resistance $R_D$ of the dropping resistor 315 is typically chosen to identical or similar to the bridge resistance RB.

If the sensor bridge 310 is supplied via the dropping resistor 315, the dropping resistor 315 and the sensor bridge 310, in combination, form a voltage divider for the output voltage $U_0$ as provided by the micro controller 305. With $R_B$ being the bridge resistance, the testing supply voltage $U_T$ is accordingly given by:

$$U_T = U_0 \cdot r$$

$$r = \frac{R_B}{R_B + R_D},$$

with r being a reduction factor. The voltage divider is substantially symmetrical if the condition $R_D = R_B$ is fulfilled. For the sensor assembly operating correctly, the differential voltage $U_D$, the output voltage $U_M$ and, thus, the output variable M are reduced by the same reduction factor r as the supply voltage $U_S$ if the supply is switched from output $OUT_1$ to output $OUT_2$. This corresponds to the expected proportional relation given by *(1)* above.

In this embodiment, the supply variable is given by the supply voltage. The operating supply level So is given by the standard supply voltage $U_0$ and the testing supply level $S_T$ is given by $r*U_0$.

In practice, the nominal resistances of the resistors 310a, 310b, 310c, 310d, that is, the resistances in the unloaded state of the sensor bridge 310, have a considerable manufacturing tolerance and, in addition, the standard supply voltage $U_0$ may not be constant over time. Therefore, the supply voltage $U_S$ is additionally measured via a second

analogue input $IN_2$ of the micro controller 105 and the measured voltage is used for the computation.

Most of the components are typically arranged on one or multiple printed circuit boards of the device. The sensor bridge 310 is connected with the other components via wiring elements such as Flexible Printed Circuit Board (Flexprint), a Flexible Cable Connector (FCC) or via single cables, as indicated by the dashed boxes 320. Those wiring elements may be assembled in a considerably bent and therefore stress-loaded state according to the overall design and the arrangements of the components of the infusion device. Alternatively, the amplifier 325 is directly arranged at the sensor bridge 310. In this case, the output of the amplifier 325 would be connected with the micro controller 305 via wiring components. Wiring components as well as soldered connections are known to be particularly susceptible to loose contacts as well as full breaks. In a design according to Figure 5, the occurrence of such a defective results in the input of the amplifier 325 floating and the measurement voltage $U_M$ as output variable of the amplifier 325 therefore not reflecting a variation of the supply voltage U as expected. Similarly, the output variable will not reflect the variation of the supply variable if any other component of the sensor assembly, in particular the amplifier 325, the sensor bridge 310 or the ADCs of the micro controller 305 show an electrical defect.

It can be seen that the embodiment of the invention as shown in Figure 5 requires only one additional hardware component, namely the dropping resistor 315. Implementation of the method for verifying correct operation of the sensor assembly according to the invention is performed by some basic computational steps which are implemented in the micro controller 305. Accordingly, correct operation of the total sensor assembly can be verified with little additional hardware and software effort.

**Reference signs**

| | |
|---|---|
| 102, 310 | sensor |
| 104 | supply unit |
| 106 | supply selector |
| 108 | testing voltage supply |
| 110 | operating voltage supply |
| 112 | measuring circuitry |
| 114 | sensor testing unit |
| 116 | controller unit |
| 120, 220 | curve of supply variable |
| 122, 122', 124, 124' | sections of supply variable curve |
| 130 | curve of output variable |
| 132, 134, 138 | sections of output variable curve |
| 252 | pulses of supply variable |
| 262a, 262b, ...262h | sampling arrow |
| 272a, 272b... 272e | sampling arrow |
| 282 | computed values |
| 305 | micro controller |
| 310a ... 310d | strain gauges |
| 311a, 311b, 312a, 312b | sensor bridge connectors |
| 315 | dropping resistor |
| 320 | wiring components |
| 325 | instrumentation amplifier |

**Claims**

1. Ambulatory infusion device for the infusion of a liquid drug into a patient's body over an extended time period, comprising:

    a) a sensor assembly (102, 112) with a sensor (102), the sensor assembly (102, 112) being configured to provide an output variable in dependence of an infusion variable and in dependence of a supply variable,
    b) a supply unit (104), the supply unit (104) being coupled to the sensor (102) and being configured to provide the supply variable,

**characterized by**

c) a sensor testing unit (114) for verifying correct operation of the sensor assembly (102, 112), the sensor testing unit (114) being coupled to the sensor assembly (102, 112) and the supply unit (104), the sensor testing unit (114) being configured to carry out a sensor testing sequence, the sensor testing sequence comprising the steps of controlling the supply unit (104) to vary the supply variable and to determine whether the variation of the supply variable is reflected by an expected variation of the output variable.

2.  Ambulatory infusion device according to Claim 1, **characterized by** the infusion variable being a drug pressure or a variable which is correlated with the drug pressure.

3.  Ambulatory infusion device according to either of the preceding claims, **characterized by** the supply unit (104) comprising a variable voltage supply (106, 108) or a variable current supply.

4.  Ambulatory infusion device according to either of the preceding claims, **characterized by** the supply unit (104) being configured to provide a set of at least two discrete levels of the supply variable.

5.  Ambulatory infusion device according to Claim 4, **characterized by** the supply unit (104) comprising at least one switchable dropping resistor (315) or shunt for varying the supply variable.

6.  Ambulatory infusion device according to either of Claim 1 to Claim 3, **characterized by** the supply unit (104) being configured to provide a substantially continuously variable supply variable.

7.  Ambulatory infusion device according to either of the preceding claims, **characterized by** the expected variation of the output variable upon variation of the supply variable being proportional to the variation of the supply variable.

8.  Ambulatory infusion device according to either of the preceding claims, **characterized by** the sensor assembly comprising a sensor with at least one force-sensitive and/or pressure-sensitive a resistor or at least one strain gauge (310a, 310b, 310c, 310d).

9.  Ambulatory infusion device according to either of the preceding claims, **characterized by** the sensor testing unit (114) being configured to measure the value of the supply variable.

10. Method for verifying correct operation of a sensor assembly (102, 112) of an ambulatory infusion device by carrying out a sensor testing sequence, the sensor assembly being configured to provide an output variable in dependence of an infusion variable and in dependence of a supply variable provided to a sensor (102) of the sensor assembly (102, 112), the sensor testing sequence comprising the steps of:

    a) varying the supply variable and
    b) determining if the variation of supply variable is reflected by an expected variation of the output variable.

11. Method as claimed in claim 10, **characterized by** the step of varying the supply variable being performed by switching the value of the supply variable between at least two discrete supply levels.

12. Method according to either of Claim 10 or Claim 11, **characterized by** the step of determining if the variation of the output variable is proportional to the variation of the supply variable.

13. Method according to either of Claim 10 to Claim 12, **characterized by** varying the supply variable such that the expected variation of the output variable is large as compared to a variation of the output variable resulting from a variation of the infusion variable while carrying out the sensor testing sequence.

14. Method according to either of Claim 10 to Claim 13, **characterized by** the sensor testing sequence being carried out in defined time intervals and/or prior to and/or during and/or after drug administration by the infusion device.

15. Method according to either of Claim 10 to Claim 14, **characterized by** the sensor comprising at least one force-sensitive and/or pressure/sensitive resistor or at least one strain gauge (310a, 310b, 310c, 310d).

**Patentansprüche**

1. Ambulante Infusionsvorrichtung zur Infusion eines flüssigen Arzneimittels in den Körper eines Patienten während eines längeren Zeitraums, umfassend:

   a) eine Sensoreinheit (102, 112) mit einem Sensor (102), wobei die Sensoreinheit (102, 112) konfiguriert ist, um eine Ausgangsgrösse in Abhängigkeit von einer Infusionsgrösse und in Abhängigkeit von einer Versorgungsgrösse bereitzustellen,

   b) eine Versorgungseinheit (104), wobei die Versorgungseinheit (104) mit dem Sensor (102) gekoppelt ist und dafür konfiguriert ist, die Versorgungsgrösse bereitzustellen,

   **gekennzeichnet durch**

   c) eine Sensortesteinheit (114) zur Überprüfung der korrekten Funktion der Sensoreinheit (102, 112), wobei die Sensortesteinheit (114) mit der Sensoreinheit (102, 112) und der Versorgungseinheit (104) gekoppelt ist, wobei die Sensortesteinheit (114) dazu konfiguriert ist, eine Sensortestsequenz durchzuführen, wobei die Sensortestsequenz die Schritte des Steuerns der Versorgungseinheit (104) umfasst, um die Versorgungsgrösse zu variieren und zu bestimmen, ob sich die Variation der Versorgungsgrösse in einer erwarteten Variation der Ausgangsgrösse widerspiegelt.

2. Ambulante Infusionsvorrichtung nach Anspruch 1, **dadurch gekennzeichnet, dass** die Infusionsgrösse ein Arzneimitteldruck oder eine Grösse ist, die mit dem Arzneimitteldruck korreliert ist.

3. Ambulante Infusionsvorrichtung nach einem der vorhergehenden Ansprüche, **dadurch gekennzeichnet, dass** die Versorgungseinheit (104) eine variable Spannungsversorgung (106, 108) oder eine variable Stromversorgung umfasst.

4. Ambulante Infusionsvorrichtung nach einem der vorhergehenden Ansprüche, **dadurch gekennzeichnet, dass** die Versorgungseinheit (104) dazu konfiguriert ist, einen Satz von mindestens zwei diskreten Niveaus der Versorgungsgrösse bereitzustellen.

5. Ambulante Infusionsvorrichtung nach Anspruch 4, **dadurch gekennzeichnet, dass** die Versorgungseinheit (104) mindestens einen schaltbaren Vorwiderstand (315) oder Shunt umfasst, um die Versorgungsgrösse zu variieren.

6. Ambulante Infusionsvorrichtung nach einem von Anspruch 1 bis Anspruch 3, **dadurch gekennzeichnet, dass** die Versorgungseinheit (104) konfiguriert ist, um eine im Wesentlichen kontinuierlich variable Versorgungsgrösse bereitzustellen.

7. Ambulante Infusionsvorrichtung nach einem der vorhergehenden Ansprüche, **dadurch gekennzeichnet, dass** die erwartete Variation der Ausgangsgrösse bei der Variation der Versorgungsgrösse proportional zur Variation der Versorgungsgrösse ist.

8. Ambulante Infusionsvorrichtung nach einem der vorhergehenden Ansprüche, **dadurch gekennzeichnet, dass** die Sensoreinheit einen Sensor mit mindestens einem kraftempfindlichen und/oder druckempfindlichen Widerstand oder mindestens einen Dehnungsmessstreifen (310a, 310b, 310c, 310d) umfasst.

9. Ambulante Infusionsvorrichtung nach einem der vorhergehenden Ansprüche, **dadurch gekennzeichnet, dass** die Sensortesteinheit (114) dazu konfiguriert ist, den Wert der Versorgungsgrösse zu messen.

10. Verfahren zur Überprüfung des korrekten Betriebs einer Sensoreinheit (102, 112) einer ambulanten Infusionsvorrichtung durch die Durchführung einer Sensortestsequenz, wobei die Sensoreinheit dazu konfiguriert ist, eine Ausgangsgrösse in Abhängigkeit von einer Infusionsgrösse und in Abhängigkeit von einer Versorgungsgrösse, welche einem Sensor (102) der Sensoreinheit (102, 112) zugeführt wird, bereitzustellen, wobei die Sensortestsequenz die folgenden Schritte umfasst:

    a) Variieren der Versorgungsgrösse, und

    b) Bestimmen, ob sich die Variation der Versorgungsgrösse in einer erwarteten Variation der Ausgangsvariablen widerspiegelt.

11. Verfahren nach Anspruch 10, **dadurch gekennzeichnet, dass** der Schritt des Variierens der Versorgungsgrösse durch die Umschaltung des Werts der Versorgungsgrösse zwischen mindestens zwei diskreten Versorgungsniveaus durchgeführt wird.

12. Verfahren nach einem von Anspruch 10 oder Anspruch 11, **gekennzeichnet durch** den Schritt des Bestimmens, ob die Variation der Ausgangsgrösse proportional zur Variation der Versorgungsgrösse ist.

13. Verfahren nach einem von Anspruch 10 bis Anspruch 12, **gekennzeichnet durch** eine derartige Variation der Versorgungsgrösse, dass die erwartete Variation der Ausgangsgrösse groß im Vergleich zu einer Variation der Ausgangsgrösse ist, die sich aus einer Variation der Infusionsgrösse ergibt, während die Sensortestsequenz durchgeführt wird.

14. Verfahren nach einem von Anspruch 10 bis Anspruch 13, **dadurch gekennzeichnet, dass** die Sensortestsequenz in definierten Zeitintervallen durchgeführt wird, und/oder vor der und/oder während der und/oder nach der Arzneimittelverabreichung durch die Infusionsvorrichtung.

15. Verfahren nach einem von Anspruch 10 bis Anspruch 14, **dadurch gekennzeichnet, dass** der Sensor mindestens einen kraftempfindlichen und/oder druckempfindlichen Widerstand oder mindestens einen Dehnungsmessstreifen (310a, 310b, 310c, 310d) umfasst.

**Revendications**

1. Dispositif de perfusion ambulatoire pour la perfusion d'un médicament liquide dans le corps d'un patient pendant une période de temps prolongée, comprenant :

   a) un ensemble capteur (102, 112) ayant un capteur (102), l'ensemble capteur (102, 112) étant configuré pour fournir une variable de sortie en fonction d'une variable de perfusion et en fonction d'une variable d'alimentation,
   b) une unité d'alimentation (104), l'unité d'alimentation (104) étant couplée au capteur (102) et étant configurée pour fournir la variable d'alimentation, **caractérisé par**
   c) une unité de test de capteur (114) pour vérifier le fonctionnement correct de l'ensemble capteur (102, 112), l'unité de test de capteur (114) étant couplée à l'ensemble capteur (102, 112) et à l'unité d'alimentation (104), l'unité de test de capteur (114) étant configurée pour réaliser une séquence de test de capteur, la séquence de test de capteur comprenant les étapes de commande de l'unité d'alimentation (114) pour faire varier la variable d'alimentation et déterminer si la variation de la variable d'alimentation est reflétée par une variation attendue de la variable de sortie.

2. Dispositif de perfusion ambulatoire selon la revendication 1, **caractérisé par le fait que** la variable de perfusion est une pression de médicament ou une variable qui est corrélée à la pression de médicament.

3. Dispositif de perfusion ambulatoire selon l'une des revendications précédentes, **caractérisé par le fait que** l'unité d'alimentation (114) comprend une alimentation en tension variable (106, 108) ou une alimentation en courant variable.

4. Dispositif de perfusion ambulatoire selon l'une des revendications précédentes, **caractérisé par le fait que** l'unité d'alimentation (114) est configurée pour fournir un ensemble d'au moins deux niveaux discrets de la variable d'alimentation.

5. Dispositif de perfusion ambulatoire selon la revendication 4, **caractérisé par le fait que** l'unité d'alimentation (114) comprend au moins une résistance chutrice commutable (315) ou shunt commutable pour faire varier la variable d'alimentation.

6. Dispositif de perfusion ambulatoire selon l'une des revendications 1 à 3, **caractérisé par le fait que** l'unité d'alimentation (114) est configurée pour fournir une variable d'alimentation variable de façon sensiblement continue.

7. Dispositif de perfusion ambulatoire selon l'une des revendications précédentes, **caractérisé par le fait que** la variation attendue de la variable de sortie lors d'une variation de la variable d'alimentation est proportionnelle à la variation de la variable d'alimentation.

**8.** Dispositif de perfusion ambulatoire selon l'une des revendications précédentes, **caractérisé par le fait que** l'ensemble capteur comprend un capteur ayant au moins une résistance sensible à la force et/ou sensible à la pression ou au moins un extensomètre (310a, 310b, 310c, 310d).

**9.** Dispositif de perfusion ambulatoire selon l'une des revendications précédentes, **caractérisé par le fait que** l'unité de test de capteur (114) est configurée pour mesurer la valeur de la variable d'alimentation.

**10.** Procédé pour vérifier le fonctionnement correct d'un ensemble capteur (102, 112) d'un dispositif de perfusion ambulatoire par réalisation d'une séquence de test de capteur, l'ensemble capteur étant configuré pour fournir une variable de sortie en fonction d'une variable de perfusion et en fonction d'une variable d'alimentation fournie à un capteur (102) de l'ensemble capteur (102, 112), la séquence de test de capteur comprenant les étapes de :

a) variation de la variable d'alimentation ; et
b) détermination du point de savoir si la variation de la variable d'alimentation est reflétée par une variation attendue de la variable de sortie.

**11.** Procédé selon la revendication 10, **caractérisé par le fait que** l'étape de variation de la variable d'alimentation est réalisée par commutation de la valeur de la variable d'alimentation entre au moins deux niveaux d'alimentation discrets.

**12.** Procédé selon l'une des revendications 10 ou 11, **caractérisé par** l'étape de détermination du point de savoir si la variation de la variable de sortie est proportionnelle à la variation de la variable d'alimentation.

**13.** Procédé selon l'une des revendications 10 à 12, **caractérisé par** la variation de la variable d'alimentation de telle sorte que la variation attendue de la variable de sortie est grande par comparaison à une variation de la variable de sortie résultant d'une variation de la variable de perfusion tout en réalisant la séquence de test de capteur.

**14.** Procédé selon l'une des revendications 10 à 13, **caractérisé par le fait que** la séquence de test de capteur est réalisée à des intervalles de temps définis et/ou avant et/ou pendant et/ou après une administration de médicament par le dispositif de perfusion.

**15.** Procédé selon l'une des revendications 10 à 14, **caractérisé par le fait que** le capteur comprend au moins une résistance sensible à la force et/ou sensible à la pression ou au moins un extensomètre (310a, 310b, 310c, 310d).

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003053498 A2
- US 20070149926 A1
- EP 1649884 A
- US 6485465 B2
- WO 200124854 A1